# EUROPEAN PATENT APPLICATION

(11) **EP 1 108 421 A2**
(43) Date of publication of application: **20.06.2001**
(21) Application number: 00310033.6
(22) Date of filing: 10.11.2000
(51) Int. Cl.: A61K 7/50

(54) **Multilayered foaming spray product**

(30) Priority: 17.12.1999 GB 9929969
(71) Applicant: UNILEVER PLC, London EC4P 4BQ (GB); UNILEVER N.V., 3013 AL Rotterdam (NL)
(72) Inventor: Lyle, Ian Gardner, 21614 Ruxtehude (DE)
(74) Representative: Rots, Maria Johanna Francisca

(57) **Abstract**

A packaged product for dispensing a foamable liquid cleansing composition in the form of a mousse comprising a sealed container having a mechanically operable dispensing means which dispenses the cleansing composition as a mousse; and a multi-phase cleansing composition in the sealed container comprising by weight of the composition (a) a first aqueous phase including from 5 to 30% by weight of the total composition of an anionic surfactant, an amphoteric surfactant, a non-ionic surfactant, a cationic surfactant, or a mixture thereof; (b) a second phase including from 10 to 80% by weight of the total composition of a water immiscible skin benefit agent, which undispensed cleansing composition separates into visually distinct phases on standing.

## Description

The present invention relates to a packaged product containing a liquid cleansing composition, in particular a packaged multilayered aqueous foamable cleansing composition including a water insoluble oil or emollient skin benefit agent for use as a shower product.

It is desirable that skin cleansing compositions not only clean the body but also have a skin caring and/or moisturising effect on the human skin. The skin caring effect is typically achieved by including an emollient/oil in the composition.

The problem of providing a shower formulation containing relatively high levels of oils which displays the required cleansing, foaming and skin caring action has long been recognised, because typically lather formation is inhibited by increasing the proportion of the water immiscible skin benefit agent in the composition.

In addition, it is also appreciated that problems may be encountered in incorporating relatively high levels of oil in aqueous compositions. A relatively high level of oil in the product is desirable so as to provide a high level of cosmetic benefit in the composition. It is also desirable that any such oil droplets should be relatively large, for example typically having a diameter in the region 0.1-0.5 mm or more, to optimise the benefit delivery to the skin. Compositions containing relatively high levels of oil benefit agent, and in addition having the oil in the.form of relatively large droplets in e.g. a course emulsion may in certain circumstances deposit relatively high levels of oil, compared to e.g. similar levels of oil in a product which is in the form of a stable emulsion, which will typically contain smaller size oil droplets. This may be because the oil in a stable emulsion with relatively small droplets may be relatively quickly washed off.

A disadvantage of such compositions having relatively high levels of oil is that they can be difficult to formulate to provide products which have a homogenous appearance. To do this requires reliance on emulsion technology, but even then the inclusion of high levels of oil into compositions can be difficult to achieve. As such, products which readily split into two visually distinct phases on standing are known.

Known products such as bath foams typically comprise an aqueous phase and a hydrophobic phase packaged in a simple container, and are intended to be shaken together just prior to dispensing to provide a course emulsion.

It is also desirable that a cleansing formulation for use in the shower does not run out of the hands after it is dispensed from a container, and is more easy to manipulate. However, when the composition includes high levels of an oil/emollient, it is more difficult to thicken the composition using conventional thickening agents, and more complicated formulations are typically employed. Commercially acceptable shower gels typically have viscosities in the region 1,000 - 10,000, more conveniently 2,000-5,000 mPa.s., measured at a shear rate of 4.5 sec⁻¹ at a temperature of 30°C.

Accordingly, there is a need for providing an improved foamable oil or skin benefit agent containing cleansing product, such as e.g. a shower oil product which overcomes the aforementioned problems of previous products.

Thus, according to a first aspect, the present invention provides a packaged product for dispensing a foamable liquid cleansing composition in the form of a mousse comprising a sealed container having a mechanically operable dispensing means which dispenses the cleansing composition as a mousse; and a multi-phase cleansing composition in the sealed container comprising by weight of the composition (a) a first aqueous phase including from 5 to 30% by weight of the total composition of an anionic surfactant, an amphoteric surfactant, a non-ionic surfactant, a cationic surfactant, or a mixture thereof; (b) a second phase including from 10 to 80% by weight of the total composition of a water immiscible skin benefit agent, which undispensed cleansing composition separates into visually distinct phases on standing.

Where percentages of compositions are quoted in this specification, all percentages are quoted as percentages by weight of the total cleansing composition, unless otherwise stated.

Conveniently the multi-phase products according to the invention have two phases, though higher numbers of phases such as e.g. three are contemplated.

By the term "multi-phase" we mean that the cleansing composition forms two or more distinct phases, which are typically visually distinct layers and which may have a visually distinct interface, after standing for a relatively short period of time. Typically phase separation occurs after a period of 5-10 minutes, though separation can take longer, such as from 30 minutes to period of several (e.g. at least 2, and possibly 20) hours. As such, the compositions can be regarded as unstable, though prior to dispensing, the multi-phase composition is shaken to provide a crude emulsion. The individual separated phases of the product can be clear or turbid.

It is also to be understood within the context of the invention that a single phase of the product may in fact itself comprise a number of different phases in the form of a stable emulsion, but if this the case, this emulsion phase will be kinetically stable, and does not separate on standing. This is in contrast to the multi-phase product of the invention, which forms a crude and unstable emulsion on shaking which is not kinetically stable, and which readily separates as described above into what may be regarded as stable individual phases of the packaged product.

Where the composition is a two phase.product, the weight ratio of the first aqueous surfactant containing phase to the second water immiscible skin benefit agent containing phase is preferably in the region of 1:4 to 4:1, and conveniently in the region 1:2 to 2:1. In certain embodiments, the preferred weight ratio between the two phases may be approximately 1:1.

This may provide the product with a particularly interesting appearance, especially if one phase contains a dye which is not soluble in the other phase, or each phase includes a different coloured dye which is not soluble in the other phase, and in either case the dispensing container is transparent or translucent (but preferably transparent).

By the term "mechanically operable", we mean that the container does not use liquifiable gas propellants, such as butane, for dispensing the composition but the container includes a pump activated dispensing means, such as a finger pump, which in use mixes air with the cleansing composition during dispensing. Alternatively, the container may be in the form of a squeeze bottle, provided the dispensed product is in the form of a mousse.

Preferably, the dispensing means mixes 1 part by volume of the composition with between about 4 and 20 parts by volume of air, more preferably about 5-12 parts of air, even more preferably 1 part of the composition with to about 5 to 8 parts of air, and delivers the cleansing composition as a mousse which may generate a foam on washing, but which can also readily be spread over the user's skin.

The dispensing means may also preferably include an integral filter mesh for increased foaming of the composition.

A suitable mechanically operable dispensing means includes the F2 Finger Pump Foamer available from Air Spray International of BV Zuiderkade 31 to 33, PO Box 389, 1940 A J Beverwijk, The Netherlands. Alternatively, a suitable squeeze bottle is illustrated in EP-A-728,475 (Kao) the contents of which (in as far as they relate to the description of squeeze bottle) are incorporated by reference.

In use, prior to dispensing the packaged product is shaken to mix the upper water immiscible skin benefit agent phase with the lower surfactant phase, thus ensuring the user receives a "fresh" mixture of surfactant and benefit agent in the form of a course emulsion. The dispensing means is then actuated, and a mousse is dispensed from the container. The mousse provides improved lathering, it has a more manipulable form to prevent egress through the fingers, and it may deliver useful quantities of the water immiscible skin benefit agent for providing the desired skin caring effect.

The composition of the present invention includes a water immiscible skin benefit agent phase for providing a skin benefit effect. The water immiscible skin benefit agent phase is typically a hydrophobic liquid such as an oil at ambient temperature, and may consist entirely of the hydrophobic liquid, or an oil in water emulsion of a water immiscible skin benefit agent, or combination of such agents. An emulsion in this context may be prepared by mechanical mixing or temporarily emulsifying the water immiscible skin benefit agent phase with the aqueous surfactant containing phase, optionally with an emulsifier to facilitate the temporary crude emulsification, or by a combination of mechanical and chemical emulsification.

Suitable water immiscible skin benefit agents include:
a) hydrocarbon oils such as mineral oil, liquid paraffin, petrolatum, polydecene, polyisobutene, squalane and squalene;
b) vegetable oils such as almond oil, arachis oil, avocado oil, castor oil, coconut oil, corn oil, cotton seed oil, evening primrose oil, jojoba oil, macadamia nut oil, olive oil, palm oil, palm kernel oil, safflower oil, rapeseed oil, sesame seed oil, sunflower seed oil, soybean oil;
c) waxes such as beeswax, carnauba wax, microcrystalline wax;
d) mono-, di- and triglycerides such as glyceryl laurate, glyceryl ricinoleate, glyceryl stearate, glyceryl isostearate, glyceryl dilaurate, glyceryl distearate, glyceryl tricaprate, glyceryl tricaprylate, glyceryl trilaurate, glyceryl trioctanoate, glyceryl trioleate, glyceryl tri(2-ethylhexanoate), glyceryl tristearate;
e) higher fatty alcohols such as lauryl, myristyl, cetyl, stearyl, isostearyl, oleyl and behenyl alcohols;
f) higher fatty acids such as lauric, myristic, palmitic, stearic, isostearic, oleic, linoleic, linolenic and behenic acids;
g) esters such as isopropyl myristate, isopropyl palmitate, isopropyl isostearate, butyl myristate, isobutyl palmitate, hexyl laurate, cetyl palmitate, isocetyl stearate, decyl oleate, myristyl lactate, cetyl lactate, octyl methoxycinnamate, propylene glycol laurate, propylene glycol stearate, propylene glycol dicaprylate, propylene glycol diisostearate; and sucrose esters;
h) silicone oils, gums and modifications thereof such as linear and cyclic polydimethylsiloxanes, amino-, alkyl-, alkylaryl- and aryl-silicones;
i) lipid materials of natural origin such as phospholipids, ceramides, cholesterol and derivatives thereof, lanolin, lanolin alcohol and derivatives thereof, lard, mink oil and tallow;
and mixtures of any of the foregoing materials.

Examples of preferred oils for use in the water immiscible skin benefit agent phase are soybean oil and/or a mineral oil.

The water immiscible skin benefit agent is generally used in an amount from about 10 to 80%, preferably 10 to 60%, optionally 20 to 50% by weight of the total composition. The water immiscible skin benefit agent may comprise a mixture of the aforementioned agents. The water immiscible skin benefit agent may also be solid at room temperature, provided it dissolves appropriately to form a solution in the hydrophobic phase. It is specifically contemplated that the water immiscible skin benefit agent may comprise up to 100% by weight of the second phase.

The water immiscible skin benefit agent may typically have a droplet size of 10-1000 microns in the crude temporary emulsion formed by shaking before use.

The composition according to the invention also comprises 5 to 30%, and preferably at least 10% by weight of the total composition of an anionic, nonionic, amphoteric or cationic surfactant or mixtures thereof.

Suitable anionic surfactants are soaps, alkyl phosphates, acyl isethionates, alkyl sulphates, alkyl ether sulphates, alkyl or aryl sulphonates, alkyl succinates, alkyl sulphosuccinates, N-alkoyl sarcosinates, and alpha-olefin sulphonates, especially their sodium, potassium, magnesium, ammonium and mono-, di- and triethanolamine salts. The alkyl groups generally contain from 8 to 18 carbon atoms and may be unsaturated. The alkyl ether sulphates may contain from one to 10 ethylene oxide or propylene oxide units per molecule, and preferably contain 2 to 3 ethylene oxide units per molecule.

Examples of suitable anionic surfactants include sodium lauryl sulphate, optionally with 2-5 EO, sodium oleyl succinate, ammonium lauryl sulphosuccinate, ammonium lauryl sulphate, sodium dodecylbenzene sulphonate, triethanolamine dodecylbenzene sulphonate and sodium N-lauryl sarcosinate. Conveniently, the anionic surfactant is chosen from sodium lauryl sulphate, sodium lauryl ether sulphate 2EO and 3EO, ammonium lauryl sulphate and/or ammonium lauryl ether sulphate 1EO, 2EO and 3EO, acyl isethionates, and alkyl phosphates. Particularly preferred embodiments of the invention may contain 5 to 30% by weight, preferably 10 to 20% by weight of the total composition of sodium lauryl ether sulphate 2 EO or 3 EO.

The nonionic surfactants suitable for use in the composition of the invention may include condensation products of aliphatic (C₈-C₁₈) primary or secondary linear or branched chain alcohols or phenols with alkylene oxides, usually ethylene oxide.

Other suitable nonionics include alkyl polyglucosides, a specific example of which is coco diglucoside. Preferably, the nonionic surfactant is present at a concentration of 0.1 to 10%, more preferably 0.1 to 3% by weight of the total composition.

The amphoteric surfactants suitable for use in the composition of the invention may include alkyl amine oxides, alkyl betaines, alkyl amidopropyl betaines, alkyl mono- or dialkanolamides, alkyl sulphobetaines, alkyl glycinates and alkyl carboxyglycinates, wherein the alkyl groups have from 8 to 18 carbon atoms. Examples include lauryl amine oxide, coco mono- or di- ethanolamide, coco mono isopropanolamide, cocamidopropyl betaine, cocodimethyl sulphopropyl betaine and coco-betaine. Particularly preferred embodiments may contain 1-10% by weight of the total composition of an amphoteric surfactant, and in particular 1-10% by weight of the total composition of cocamidopropylbetaine.

Conveniently the composition may contain up to about 50 wt%, more preferably 5 to 30 wt% of the total composition of a material which may act to increase the density of the aqueous (surfactant containing) phase. Suitable materials include short chain polyhydric alcohols such as glycerol or sorbitol, or polyethylene glycols (typically having a MW of 1000 to 100,000). These have the effect of increasing the density difference between the aqueous and oil/emollient phases, thereby facilitating the separation between the two phases on standing, and may also contribute some humectant properties to the composition.

Compositions according to the invention also contain water, typically at levels of 10-75 wt%, more preferably 20-60 wt% of the total composition.

The cleansing composition may also include minor amounts of other ingredients such as a perfume, a colouring agent, a preservative, a thickener, an antioxidant or mixtures thereof, typically in amounts of from 0 to 5% by weight of the total composition.

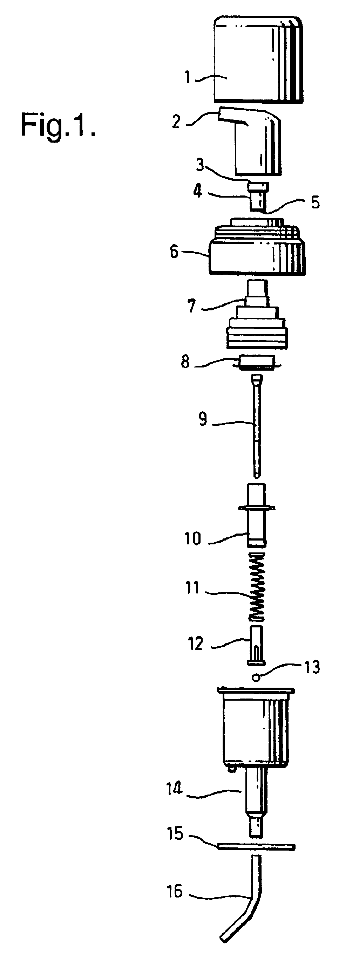

The invention is further illustrated by way of example only with reference to the following drawing and examples, in which figure 1 represents an exploded drawing of suitable mechanically operable spray device for use in compositions according to the invention.

Figure 1 shows a spray device (F2 Finger Pump Foamer, ex. Air Spray International) which is conveniently and readily attached to a supply container (not shown), which is preferably transparent, containing the composition to be dispensed. Spray device contains components 1-16 which combine as indicated in the exploded diagram, and co-operate in the conventional manner to dispense liquid contained in the supply container in the form of a mousse.

Components shown in figure 1 are;
1. Polypropylene over cap
2. Polypropylene nozzle
3. PET mesh 2
4. Polypropylene netholder
5. PET mesh 1
6. Polypropylene base cap
7. High density polyethylene air piston
8. Low density polyethylene blend valve
9. Polypropylene inner rod
10. High density polyethylene liquid piston
11. Stainless steel spring
12. Polypropylene plug
13. Stainless steel ball
14. Polypropylene cylinder
15. Tri-seal gasket
16. Polyethylene dip tube

Particularly significant features of this embodiment of spray dispenser are the meshes 3 and 5, which when the liquid composition is forced through them generate a fine porous foam.

Preferred hardware for use in products according to the invention has a volume ratio of product:air of approximately 1:5 to 1:12, more preferably about 1:5 to 1:8.

A suitable composition which forms a crude emulsion on shaking, but otherwise separates into two visibly distinct phases on standing, is as follows;

| Component | %wt |
|---|---|
| Sodium laureth sulphate | 15.0 |
| Cocoamidopropyl betaine | 3.0 |
| Citric acid | 0.2 |
| PEG-120 methyl glucose dioleate | 0.2 |
| Sodium benzoate | 0.4 |
| Water soluble dye | 0.02 |
| Perfume | 0.8 |
| Soyabean oil | 12.5 |
| Mineral oil | 12.5 |
| Water | to 100 |

## Claims

**1.** A packaged product for dispensing a foamable liquid cleansing composition in the form of a mousse comprising a sealed container having a mechanically operable dispensing means which dispenses the cleansing composition as a mousse; and a multi-phase cleansing composition in the sealed container comprising by weight of the composition (a) a first aqueous phase comprising from 5 to 30% by weight of the total composition of an anionic surfactant, an amphoteric surfactant, a non-ionic surfactant, a cationic surfactant, or a mixture thereof; (b) a second phase comprising from 10 to 80% by weight of the total composition of a water immiscible skin benefit agent, which undispensed cleansing composition separates into visually distinct phases on standing.

**2.** A packaged product as claimed in Claim 1 wherein the multiphase product is a two phase product.

**3.** A packaged product as claimed in Claim 1 or Claim 2 wherein the anionic surfactant is chosen from sodium lauryl ether sulphate 2EO, sodium lauryl ether sulphate 3EO, ammonium lauryl sulphate, ammonium lauryl ether sulphate 1EO, ammonium lauryl ether sulphate 2EO, ammonium lauryl ether sulphate 3EO, acyl isethionates, alkyl phosphates, or mixtures thereof.

**4.** A packaged product as claimed in Claim 3 wherein the composition includes 5 to 30% by weight, preferably 10 to 20% by weight, of sodium lauryl ether sulphate 2EO.

**5.** A packaged product as claimed in any one of the preceding claims wherein the amphoteric surfactant is a C₈-C₁₈ alkyl amidopropylbetaine, a C₈-C₁₈ alkyl betaine, a C₈-C₁₈ alkyl mono- or di-alkanolamide, or a mixture thereof.

**6.** A packaged product as claimed in Claim 5 wherein the composition includes 0.1 to 20% by weight, preferably 1 to 10% by weight, of cocamidopropyl betaine.

**7.** A packaged product as claimed in any one of the preceding claims wherein the composition contains a nonionic surfactant.

**8.** A packaged product as claimed in Claim 7 wherein the composition includes 0.1 to 10% by weight, preferably 0.1 to 3% by weight, of a nonionic surfactant.

**9.** A packaged product as claimed in any one of the preceding claims wherein the water immiscible skin benefit agent is a vegetable oil, a mineral oil, a silicone oil, an animal oil, a triglyceride of a fatty acid and/or a fatty alcohol, an ester of a fatty acid and/or a fatty alcohol, or a mixture thereof.

**10.** A packaged product as claimed in Claim 9 wherein the composition includes 5 to 80% by weight, preferably 10 to 50% by weight, of a soybean oil, a mineral oil, or a mixture thereof.

**11.** A packaged product as claimed in any one of the preceding claims wherein the composition can be shaken to form a crude emulsion prior to use.

**12.** A packaged product according to claim 11, wherein the crude emulsion has a particle size of 10-1000 microns.

**13.** A packaged product according to claim 11 or claim 12, wherein the crude emulsion separates on standing into two distinctive layers within a period of 2 hours.

**14.** A packaged product as claimed in any one of the preceding claims wherein the second phase containing the water immiscible skin benefit agent phase comprises one third to two thirds by weight of the packaged composition.

**15.** A packaged product according to any of the preceding claims, wherein the composition contains a dye which is soluble in one phase but not the other(s).

**16.** A packaged product according to any of claims 1-15, wherein the composition comprises two dyes, each being soluble in one phase but not the other(s).

**17.** A packaged product as claimed in any one of the preceding claims wherein the composition further includes 0 to 5% by weight of a perfume, a colouring agent, a preservative, a thickener, an antioxidant, or a mixture thereof.

**18.** A packaged product as claimed in any one of the preceding claims comprising 20-60 wt% water.

**19.** A packaged product as claimed in any one of the preceding claims wherein the composition is packaged in a transparent or translucent container.

**20.** A packaged product according to any of the preceding claims, wherein the aqueous phase contains a material which increases the density of the aqueous phase.

**21.** A packaged product according to claim 21, wherein the composition contains up to 50 wt% glycerol, polyethylene glycol or sorbitol.

**23.** A method of dispensing a foamable liquid cleansing composition comprising: providing a sealed container having a mechanically operable dispensing means and containing a two-phase cleansing composition as claimed in any one of the preceding claims in the container; and mechanically operating the dispensing means.
